# EUROPEAN PATENT SPECIFICATION

(11) **EP 0 355 848 B1**
(45) Date of publication and mention of the grant of the patent: **23.03.1994**
(21) Application number: 89115726.5
(22) Date of filing: 25.08.1989
(51) Int. Cl.: C11D 3/24, C11D 17/00

(54) **Syndet soap bars having improved properties**
Synthetische Seifenstücke mit verbesserten Eigenschaften
Barres de savon synthétiques ayant des propriétés améliorées

(30) Priority: 26.08.1988 IT 2175688
(43) Date of publication of application: 28.02.1990
(73) Proprietor: AUSIMONT S.p.A., I-20121 Milano (IT)
(72) Inventor: Pantini, Giovanni, Dr., I-20121 Milan (IT); Savonelli, Susanna, Dr., I-27100 Voghera Pavia (IT)
(74) Representative: Barz, Peter, Dr.

(56) References cited:
- DE-B- 1 002 492
- GB-A- 2 106 928
- US-A- 3 976 601
- US-A- 4 409 118

## Description

The present invention relates to syndet soap bars having improved properties.

It is known that syndet soap bars, also called "syndets" or "non-soap soaps", are based on solid synthetic detergents.

As compared to the soap cakes manufactured from natural soap, they show several advantages. However, their use and manufacturing suffer from drawbacks. The first drawback is the formation of clefts (cracks) in the soap bars during their use; besides being disadvantageous from an aesthetical point of view, this alteration may lead to a completely broken product.

Secondly, the preparation of the syndet soap bars, which is carried out on facilities designed for the processing of relatively flexible products, such as natural soap, is difficult, owing to the higher stiffness of syndet soaps.

One object of the present invention is to provide syndet soaps which do not or only to a limited extent show cracking phenomena.
A further object of the present invention is to facilitate the preparation of syndet soap bars.

It has now surprisingly been found that the addition to the syndet formulation of small amounts of perfluoropolyethers having perfluoroalkyl end groups eliminates or strongly reduces the phenomena of soap bar cleaving (cracking) and facilitates the preparation of said soap bars in that it secures a greater ease of homogenization of the composition in paste form, and makes it easier to be extruded and moulded.

Therefore, the present invention provides syndet soap bars containing, in addition to the usual components of said soap cakes, from 0.001 to 10% by weight of one or more perfluoropolyethers having perfluoroalkyl end groups.

The perfluoropolyethers having perfluoroalkyl end groups, i.e. without functional groups, are well-known compounds which are described, together with corresponding methods of preparation, in various documents, e.g., in GB-A-1,104,482; US-A-3,242,218; 3,665,041; 3,715,378 and 4,532 039; EP-A-148,482, 151,877 and 191,490 and in international patent applications WO-A-87/00538 and WO-A-87/02992.

Examples of suitable perfluoropolyethers are those which are characterized by the presence of one or more perfluorooxyalkylene units selected from
a) (CF₂-CF₂O),
b) (CF₂O)
c) (C₃F₆O), a simplified formula for 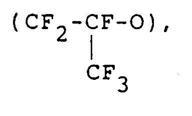
d) (CF₂O-CF₂-CF₂O),
e) (CF₂-CF₂-CF₂O),
f) 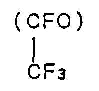 and
g) 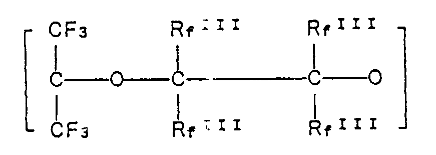
wherein the groups R_{f}III, the same or different from each other, are fluorine atoms or (preferably C₁₋₃-) perfluoroalkyl groups (e.g. CF₃, C₂F₅ and C₃F₆).

Perfluoropolyethers suitable for use in the present invention preferably contain one or more of the following individual perfluorooxyalkylene units or combinations of perfluorooxyalkylene units:
(I) (CF₂-CF₂O) and (CF₂O), said units being randomly distributed along the perfluoropolyether chain;
(II) 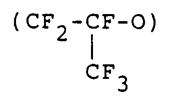 and (CFXO), wherein X is F or CF₃, said units being randomly distributed along the perfluoropolyether chain;
(III) (CF₂-CF₂O), 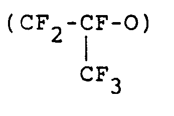 and (CFXO),
   wherein X is F or CF₃, said units being randomly distributed along the polyfluoropolyether chain;
(IV) 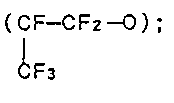
(V) (CF₂-CF₂-CF₂O);
(VI) (CF₂-CF₂O);
(VII) 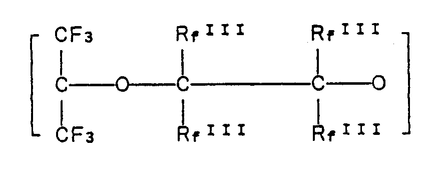 wherein the groups R_{f}III, the same or different from each other, are fluorine atoms or (preferably C₁₋₃-) perfluoroalkyl groups; and
(VIII) (CF₂O-CF₂-CF₂O).

Also suitable are perfluoropolyethers which contain perfluorooxetane rings of formula 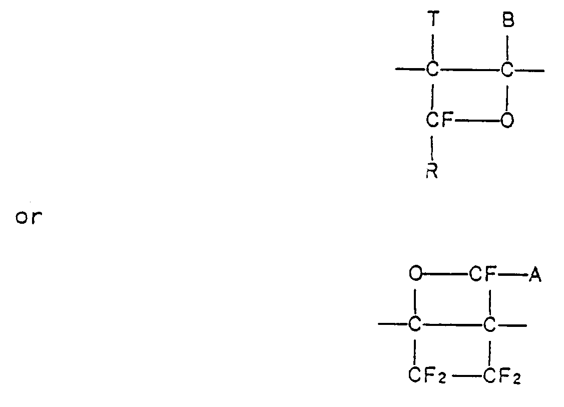
wherein
T, B and R, the same or different from each other, are perfluorooxyalkyl, perfluoropolyoxyalkyl or perfluoroalkyl radicals, and
A is a perfluorooxyalkyl, perfluoropolyoxyalkyl or perfluoroalkyl radical.

Examples of perfluoroalkyl radicals are CF₃, C₂F₅ and C₃F₉ while examplary perfluorooxyalkyl radicals are those given above under (a) to (g), terminated with a F atom. Suitable perfluoropolyoxyalkyl radicals include those composed of one or more units given above under (a) to (g), also terminated by a F atom.

Examples of suitable perfluoropolyethers containing perfluorooxyalkylene units are those belonging to the following classes:
A) 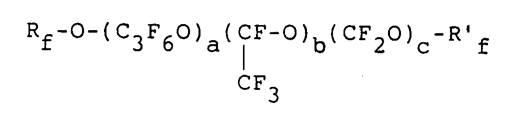 wherein:
   - R_{f} and R′_{f},: the same or different from each other, are selected from CF₃, C₂F₅ and C₃F₇;
   the units in brackets are randomly distributed along the polyether chain;
   - a: is an integer; and
   - b and c: represent integers or zero; the ratio a/(b+c) ranging from 5 to 40 when the sum (b+c) is different from zero;
B) CF₃O-(C₂F₄O)_{d}(CF₂O)ₑ-CF₃
   wherein the C₂F₄O and CF₂O units are randomly distributed along the polyether chain; d and e are integers; and the ratio d/e ranges from 0.3 to 5;
C) CF₃O-(C₃F₆O)_{f}(C₂F₄O)_{g}(CFXO)ₕ-CF₃
   wherein the C₃F₆O, C₂F₄O and CFXO units are randomly distributed along the polyether chain;
   X is F or CF₃;
   f, g and h are integers;
   the ratio f/(g+h) ranges from 1 to 50; and
   the ratio g/h ranges from 1 to 10;
D) R³_{f}O-(CF₂CF₂CF₂O)ⱼR⁴_{f}
   wherein R³_{f} and R⁴_{f}, the same or different from each other, represent -CF₃ or -C₂F₅ and j is an integer.

The perfluoropolyethers suitable for use in the present invention preferably have an average molecular weight of from 800 to 20,000, and, even more preferred of from 1,500 to 10,000.

The concentration of perfluoropolyether in the formulation preferably ranges from 0.1 to 2% by weight.

The perfluoropolyethers usually are added to the syndet formulation before the latter is homogenized.

Syndet soap bars may be prepared as follows: As the starting formulation, a syndet base composition in powder form is used. Said base composition is mixed with water, perfluoropolyethers and other usual additives, and then the whole mixture is homogenized. The product is extruded in the form of rods, which rods are cut whereafter the syndet soap bars are moulded on an automatic press.

The perfluoropolyethers employed according to the present invention show a perfect compatibility with the other components of the soap bar, and do not modify the physico-chemical characteristics of the aqueous solutions of syndet soap bars to any significant extent.

As the remaining components of the syndet soap bars according to the present invention all of the usual components of said soap bars can be used.

For example, as solid surfactants the following products may be employed:
a′) isethionates, i.e., condensation products of a fatty acid of from 12 to 18 carbon atoms with sodium isethionate;
b′) taurates, i.e., N-methyl-taurine amides of fatty acids of from 12 to 18 carbon atoms;
c′) sulfates of fatty alcohols of from 12 to 18 carbon atoms;
d′)sulfosuccinates of fatty alcohols of from 12 to 18 carbon atoms.

The preferred organic acids used in order to adjust the pH of the aqueous solution of the soap bar at the desired value, are lactic acid and stearic acid.

All of the common plasticizers, such as, e.g., fatty acids, esters and alkanolamides thereof, fatty alcohols and paraffins, can be also used. Conventional fillers, such as polysaccharides and talc, can be used as well.

Pigments, perfumes and active principles may also be added.

The addition of the perfluoropolyethers according to the present invention considerably improves the properties of the syndet soap bars in that the resulting soap bars do not show the phenomenon of cracking during use or, at least, show said phenomenon to a very limited extent only.

Moreover, in the preparation of soap bars, the paste-like composition is easier to homogenize, and can easier be extruded and moulded.

The following examples are given in order to illustrate the present invention without, however, limiting it.

### EXAMPLE 1

Several samples of syndet soap bars were prepared, which samples contained perfluoropolyethers according to the present invention (samples 2, 3, 5 and 6). For comparative purposes, syndet soap bars which did not contain any perfluoropolyethers (samples 1 and 4 ), were also prepared.

The following perfluoropolyethers, manufactured by Montefluos, were used:
a) Fomblin® HC/04, having an average molecular weight of about 1,500 and a viscosity of 35 cSt at 20°C;
b) Fomblin® HC/25, having an average molecular weight of about 3,200 and a viscosity of 250 cSt at 20°C;
c) Fomblin® HC/R, having an average molecular weight of about 6,600 and a viscosity of 1,500 cSt at 20°C.

All of the above commercial products may be represented by the formula: 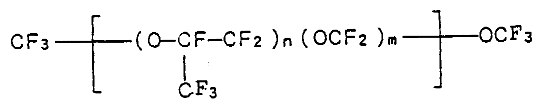
wherein n/m is from 20 to 40.

Two types of syndet base compositions were used:
1. A composition containing from 80 to 87% of sodium (coconut acid) isethionate;
2. Zetesap® 813A, by Zchimmer and Schwarz, containing the following components:

| | |
|---|---|
| combination of sulfates and sulfo-succinates of fatty alcohols of from 12 to 18 carbon atoms | 50% b.w. |
| polysaccharides | 23% b.w. |
| plasticizers | 23% b.w. |
| water and other ingredients | up to 100% b.w. |

Six samples (1 to 6) were prepared; their composition is shown in Table 1 below.

On a 5% b.w. aqueous solution of each of said samples the conductivity X, the pH value and the surface tension γsup were determined at 25°C. The obtained results are reported in Table 1.

A comparison of the samples with and without various Fomblin® HC grades shows that the addition of the perfluoropolyether to the syndet soap bar formulation does not modify the physico-chemical characteristics thereof to any significant extent.

**TABLE 1**

| Sample Number | Composition | | X (mS) | pH | γsup (dyne/cm) |
|---|---|---|---|---|---|
| 1 | Zetesap® 813 A | 100 % | 3 | 7.10 | 30.3 |
| 2 | Zetesap® 813 A | 99.5% | 3.4 | 7.10 | 32.4 |
| | Fomblin® HC/25 | 0.5% | | | |
| 3 | Zetesap® 813 A | 98 % | 2.9 | 6.55 | 31.0 |
| | Fomblin® HC/25 | 1 % | | | |
| | Perfume | 1 % | | | |
| 4 | Sodium coconut acid | | 3.2 | 6.63 | 30.2 |
| | isethionate (80-87%) | 50 % | | | |
| | Zetesap® 813 A | 50 % | | | |
| 5 | Sodium coconut acid | | 3 | 6.51 | 30.3 |
| | isethionate (80-87%) | 50 % | | | |
| | Zetesap® 813 A | 49 % | | | |
| | Fomblin® HC/25 | 1 % | | | |
| 6 | Sodium coconut acid | | 2.9 | 5.80 | 31.0 |
| | isethionate (80-87%) | 50 % | | | |
| | Zetesap® 813 A | 49.4% | | | |
| | Fomblin® HC/25 | 0.4% | | | |
| | Fomblin® HC/R | 0.2% | | | |

### EXAMPLE 2

The present example describes a comparative test with respect to accelerated ageing during use of a syndet soap bar containing Fomblin® HC/25 (sample 2 of example 1) and of a similar syndet soap bar not containing any perfluoropolyethers(sample 1 of example 1).

The accelerated ageing of the soap cakes was simulated by holding said soap bars (weight about 25 g) under running tap water for 4 hours at room temperature and then leaving them standing for 24 hours in a ventilated oven at 30°C. Said treatment was repeated over 8 days.

After the above treatment, a significant difference was observed between the two tested samples. On the surface of the reference sample a larger number of more visible alterations was observed: bubbles, slight or small-size but deep clefts or cracks; said reference soap bar additionally was affected by large cracks at its edges. Furthermore, by measuring the weight loss, it was determined that a larger amount of the reference soap bar had been dissolved: in fact, 64% of the reference sample had dissolved, as compared to 49% of the sample which contained Fomblin® HC/25.

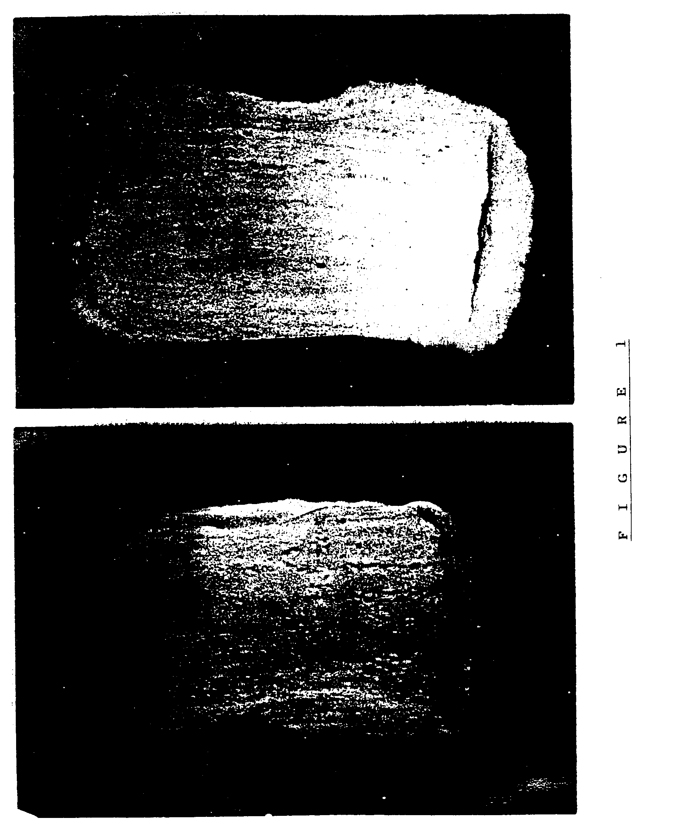

A photograph of the soap bars after the test, at a magnification of 2.5X, is shown in Figure 1. The right-hand side soap bar is the one which contained Fomblin® HC/25; the left-hand soap bar is the reference sample which did not contain perfluoropolyether.

The above mentioned alterations can be observed in the reference sample.

### EXAMPLE 3

The present example relates to an accelerated ageing test wherein a syndet soap bar containing Fomblin® HC/25 (sample 7) and a similar (comparative) syndet soap bar not containing any perfluoropolyether (sample 8) were employed.

Sample 7 had the following composition:

| | |
|---|---|
| - sodium coconut acid isethionate (80-87%) | 17 parts b.w. |
| - fats, plasticizers and binders | 78 parts b.w. |
| - Fomblin® HC/25 | 0.5 parts b.w. |
| - water | 5 parts b.w. |

Sample 8 had the following composition:

| | |
|---|---|
| - sodium coconut acid isethionate (80-87%) | 17 parts b.w. |
| - fats, plasticizers and binders (the same as in sample 7) | 78 parts b.w. |
| - water | 5 parts b.w. |

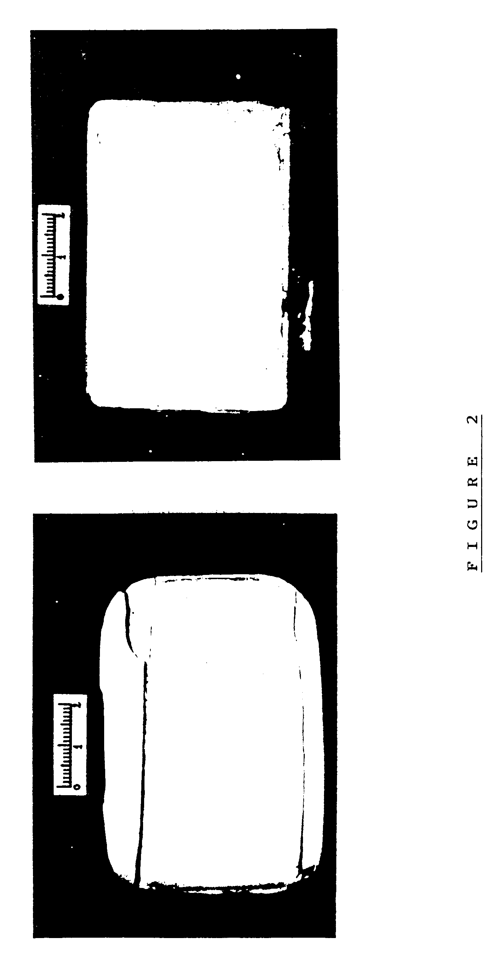

An accelerated ageing was simulated on the two bars (weight about 75 g) by holding them under running tap water for 4 hours at room temperature and then leaving them standing for 24 hours in a ventilated oven at 30°C; said treatment was repeated over 8 days. A photograph of the bars after the test is shown in Figure 2. The bar on the right is the one containing Fomblin® HC/25, the bar on the left is the comparison sample not containing perfluoropolyether. Big cracks are present in the latter sample.

## Claims

1. Syndet soap bars characterized in that they contain, in addition to the usual components of said soap bars, from 0.001 to 10% by weight of one or more perfluoropolyethers having perfluoroalkyl end groups.

2. Syndet soap bars according to claim 1, characterized in that the perfluoropolyethers contain one or more perfluorooxyalkylene units selected from:
a) (CF₂-CF₂O)
b) (CF₂O)
c) (C₃F₆O)
d) (CF₂O-CF₂-CF₂O)
e) (CF₂-CF₂-CF₂O)
f) 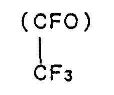 and
g) 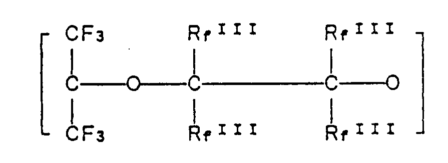 wherein the groups R_{f}III, the same or different from each other, represent fluorine atoms or perfluoroalkyl groups.

3. Syndet soap bars according to any one of claims 1 and 2, characterized in that the perfluoropolyethers contain one or more individual perfluorooxyalkylene units or combinations of perfluorooxyalkylene units selected from:
I) (CF₂-CF₂O) and (CF₂O), said units being randomly distributed along the perfluoropolyether chain;
II) 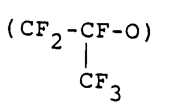 and (CFXO), wherein X is F or CF₃, said units being randomly distributed along the perfluoropolyether chain;
III) (CF₂-CF₂O), 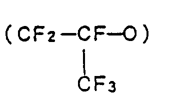 and (CFXO),
wherein X is F or CF₃, said units being randomly distributed along the perfluoropolyether chain;
IV) 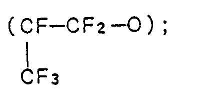
V) (CF₂-CF₂-CF₂O);
VI) (CF₂-CF₂O);
VII) 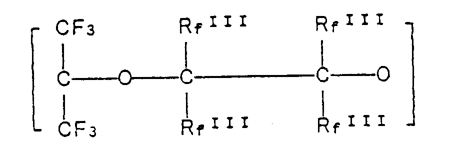 wherein the groups R_{f}III, the same or different from each other, are fluorine atoms or perfluoroalkyl groups; and
VIII) (CF₂O-CF₂-CF₂O).

4. Syndet soap bars according to any one of claims 1 to 3 characterized in that the perfluoropolyethers contain perfluorooxetane rings of formula: 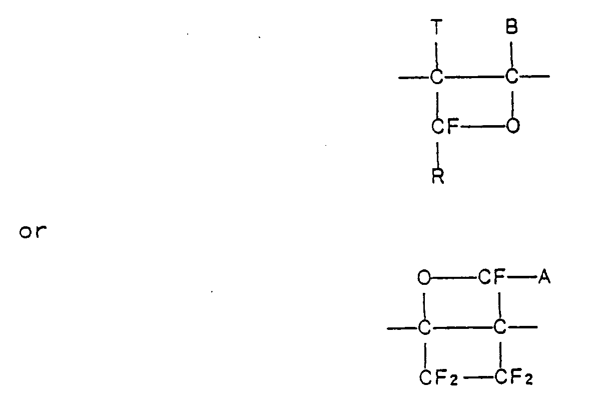 wherein:
T, B and R, the same or different from each other, are perfluorooxyalkyl, perfluoropolyoxyalkyl or perfluoroalkyl radicals, and
A is a perfluorooxyalkyl, perfluoropolyoxyalkyl or perfluoroalkyl radical.

5. Syndet soap bars according to one or more of the preceding claims, characterized in that the perfluoroalkylpolyethers have an average molecular weight of from 800 to 20,000.

## Patentansprüche

1. Syndet-Seifenstücke, dadurch gekennzeichnet, daß sie zusätzlich zu den üblichen Komponenten von Seifenstücken 0,001 bis 10 Gewichtsprozent eines oder mehrerer Perfluorpolyether mit Perfluoralkyl-Endgruppen enthalten.

2. Syndet-Seifenstücke nach Anspruch 1, dadurch gekennzeichnet, daß die Perfluorpolyether eine oder mehrere Perfluoroxyalkylen-Einheiten enthalten, die ausgewählt sind aus:
a) (CF₂-CF₂O)
b) (CF₂O)
c) (C₃F₆O)
d) (CF₂O-CF₂-CF₂O)
e) (CF₂-CF₂-CF₂O)
f) 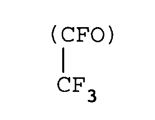 und
g) 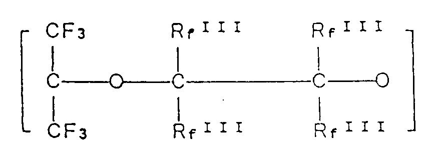 worin die Gruppen R_{f}III, gleich oder verschieden voneinander, Fluoratome oder Perfluoralkylgruppen darstellen.

3. Syndet-Seifenstücke nach irgendeinem der Ansprüche 1 und 2, dadurch gekennzeichnet, daß die Perfluorpolyether eine oder mehrere einzelne Perfluoroxyalkylen-Einheiten oder Kombinationen von Perfluoroxyalkylen-Einheiten enthalten, die ausgewählt sind aus:
I) (CF₂-CF₂O) und (CF₂O), wobei diese Einheiten statistisch entlang der Perfluorpolyether-Kette verteilt sind;
II) 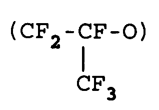 und (CFXO), worin X für F oder CF₃ steht, wobei diese Einheiten statistisch entlang der Perfluorpolyether-Kette verteilt sind;
III) (CF₂-CF₂O), 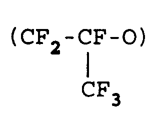 und (CFXO),
worin X für F oder CF₃ steht, wobei diese Einheiten statistisch entlang der Perfluorpolyether-Kette verteilt sind;
IV) 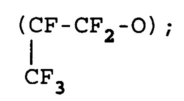
V) (CF₂-CF₂-CF₂O);
VI) (CF₂-CF₂O);
VII) 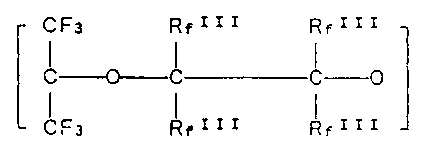 worin die Gruppen R_{f}III, gleich oder verschieden voneinander, Fluoratome oder Perfluoralkylgruppen sind; und
VIII) (CF₂O-CF₂-CF₂O).

4. Syndet-Seifenstücke nach irgendeinem der Ansprüche 1 bis 3, dadurch gekennzeichnet, daß die Perfluorpolyether Perfluoroxetan-Ringe der Formel: 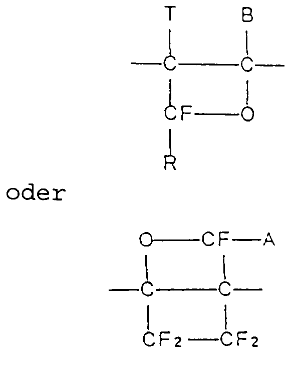 enthalten, worin :
T, B und R, gleich oder verschieden voneinander, Perfluoroxyalkyl-, Perfluorpolyoxyalkyl- oder Perfluoralkyl-Reste sind und
A ein Perfluoroxyalkyl-, Perfluorpolyoxyalkyl- oder Perfluoralkyl-Rest ist.

5. Syndet-Seifenstücke nach einem oder mehreren der vorangehenden Ansprüche, dadurch gekennzeichnet, daß die Perfluoralkylpolyether ein durchschnittliches Molekulargewicht von 800 bis 20000 aufweisen.

## Revendications

1. Barres de savon synthétique caractérisées en ce qu'elles contiennent, outre les composants habituels de ces barres de savon, de 0,001 à 10% en poids d'un ou de plusieurs perfluoropolyéthers renfermant des groupes terminaux perfluoroalkyl.

2. Barres de savon synthétique selon la revendication 1, caractérisées en ce que les perfluoropolyéthers contiennent un ou plusieurs motifs perfluorooxyalkylène choisis parmi:
a) (CF₂-CF₂O),
b) (CF₂O)
c) (C₃F₆O)
d) (CF₂O-CF₂-CF₂O),
e) (CF₂-CF₂-CF₂O),
f) 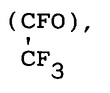 et
g) 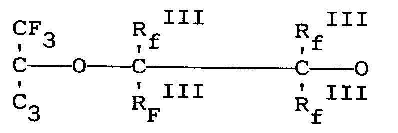 où les groupes R_{F}^{III}, identiques ou différents les uns des autres, représentent des atomes de fluor ou des groupes perfluoroalkyl.

3. Barres de savon synthétique selon l'une quelconque des revendications 1 et 2, caractérisées en ce que les perfluoropolyéthers contiennent un ou plusieurs motifs perfluorooxyalkylène suivants, ou des associations de motifs perfluoroalkylène choisis parmi:
(1) (CF₂-CF₂O) et (CF₂O), ces motifs étant distribués statistiquement le long de la chaîne perfluoropolyéther;
(2) 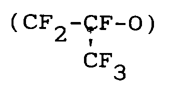 et (CFXO) dans lequel X représente F ou CF₃, ces motifs étant distribués statistiquement le long de la chaîne perfluoropolyéther;
(3) (CF₂-CF₂O), 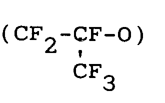 et (CFXO),
dans lequel X représente F ou CF₃, ces motifs étant distribués statistiquement le long de la chaîne perfluoropolyéther;
(4) 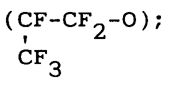
(5) (CF₂-CF₂-CF₂O);
(6) (CF₂-CF₂O);
(7) 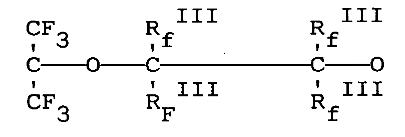 dans lequel les groupes R_{f}^{III} identiques ou différents les uns des autres sont des atomes de fluor ou des groupes perfluoroalkyle; et
(8) (CF₂O-CF₂-CF₂O).

4. Barres de savon synthétique selon l'une quelconque des revendications 1 à 3 , caractérisées en ce que les perfluoropolyéthers contiennent des cycles perfluorooxétane de formule: 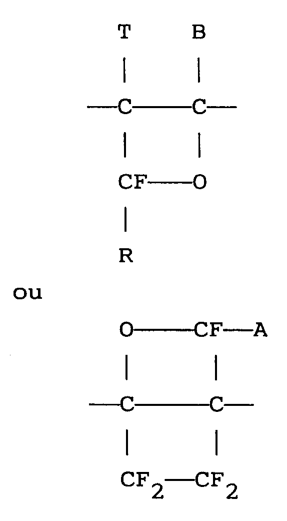 dans lesquelles
T, B et R, identiques ou différents les uns des autres, sont des radicaux perfluorooxyalkyl, perfluoropolyoxyalkyl, et perfluoroalkyl, et
A est un radical perfluorooxyalkyl, perfluoropolyoxyalkyl ou perfluoroalkyl.

5. Barres de savon synthétique selon l'une ou plusieurs des revendications précédentes, caractérisées en ce que le poids moléculaire moyen des perfluoroalkylpolyéthers est compris entre 800 et 20000.
